(19) Europäisches Patentamt
European Patent Office
Office européen des brevets

(11) **EP 4 736 779 A1**

(12) **EUROPEAN PATENT APPLICATION**

(43) Date of publication:
**06.05.2026  Bulletin 2026/19**

(21) Application number: **24210947.8**

(22) Date of filing: **05.11.2024**

(51) International Patent Classification (IPC):
**A61B 8/06** (2006.01)      **A61B 8/08** (2006.01)
**A61B 8/00** (2006.01)      **A61B 8/04** (2006.01)

(52) Cooperative Patent Classification (CPC):
**A61B 8/06; A61B 8/0891; A61B 8/5223;**
A61B 8/04; A61B 8/488

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **LAU, Kevin Daniel Seng Hung**
**5656 AG Eindhoven (NL)**
• **JOSHI, Rohan**
**5656 AG Eindhoven (NL)**
• **DINIS FERNANDES-KUILBOER, Catarina**
**5656 AG Eindhoven (NL)**

(74) Representative: **Philips Intellectual Property & Standards**
**High Tech Campus 52**
**5656 AG Eindhoven (NL)**

(54) **HEMODYNAMIC MONITORING SYSTEM**

(57)    A method for evaluating changes in patient hemodynamic status, e.g. hemodynamic responsiveness, between before and after clinical intervention events. Waveform features extracted from an ensemble average blood velocity pulse waveform for the patient are fed as input to an analysis algorithm, for example comprising a classifier, to generate an output indicative of one or more hemodynamic characteristics, for example indicative of hemodynamic responsiveness. This can be used to evaluate changes in hemodynamic responsiveness before and after a clinical intervention event.

FIG. 2

**Description**

FIELD OF THE INVENTION

The present invention pertains to the field of medical technology, specifically to systems and methods for monitoring a patient's hemodynamic status using ultrasound data.

BACKGROUND OF THE INVENTION

Hemodynamic monitoring is an important aspect of patient monitoring.
Hemodynamics refers to the physical principles governing the circulation of blood in the cardiovascular system. It involves inter alia the assessment of blood volume, pressure, and the oxygenation level in the blood. Monitoring these parameters is of significance in the management of patients in intensive care units (ICUs) and during high-risk surgeries.
One aspect of hemodynamic monitoring is the assessment of fluid responsiveness. Fluid responsiveness is a term used to describe the likelihood that a patient's cardiac output will increase in response to fluid administration. Accurate assessment of fluid responsiveness is of particular clinical relevance, especially in the management of patients with circulatory failure. A measure of fluid responsiveness can help guide clinicians in deciding whether to administer fluids to improve physiological stability and patient outcome.
Monitoring of fluid responsiveness in the current state of the art involves invasive monitoring methods such as an arterial catheter. Non-invasive methods for monitoring fluid responsiveness, and hemodynamic status more generally, would be of value.

SUMMARY OF THE INVENTION

The invention is defined by the claims.
In accordance with an aspect of the invention, a computer-implemented method is provided. The method includes receiving first and second ultrasound data of a blood vessel of a subject, the first ultrasound data acquired over a first epoch occurring prior to a clinical intervention event or patient interaction event, and the second ultrasound data acquired over a second epoch occurring following the clinical intervention event or patient interaction event. The method further includes deriving from the received first and second ultrasound data first and second blood velocity-time signals representative of blood velocity over a time window contained in the first and second epochs respectively. The method further includes computing first and second ensemble average blood velocity waveforms from a plurality of blood velocity waveforms present in the first and second blood velocity-time signals respectively. Each respective one of the plurality of blood velocity waveforms may correspond to a respective single respective heartbeat. The method further includes determining first and second sets of parameters by extracting from the first and second ensemble average blood velocity waveforms one or more pre-defined blood velocity waveform features. The method also includes retrieving and applying a pre-defined analysis algorithm configured to receive the first and second sets of parameters as inputs and to generate a hemodynamic response indicator as an output, the hemodynamic response indicator indicative of characteristics of a patient's hemodynamic responsiveness to the clinical intervention event.
In the context of the present disclosure, a "clinical intervention event" may refer to any intervention performed in relation to a patient, for example a medical procedure or treatment, for example an intervention for modifying the patient's physiological status. The clinical intervention events could for example include any event that could potentially affect the patient's hemodynamic status, such as a change in medication, a surgical procedure, or a change in the patient's physical activity level. Examples of clinical intervention events include, but are not limited to, end-expiratory occlusion pressure (test), inspiratory hold positive end-expiratory pressure 10cm H2O, inspiratory hold positive end-expiratory pressure 15cm H2O, a passive leg raise, and intra-arterial administration of a bolus of liquid to the subject. Other examples of clinical intervention events include any kind of surgical procedure.
In some embodiments, the method includes processing the blood velocity-time signal to detect and/or extract the said plurality of blood velocity waveforms from the signal. From these, the ensemble average blood velocity waveform can be computed, for example by aligning and then averaging the plurality of extracted blood velocity waveforms. Each blood velocity waveform corresponds to a heart beat and is a pulse in the blood velocity at the measurement site at which the relevant ultrasound data is acquired.
The analysis algorithm used in the method can take various forms. For example, it could be a multiparametric regression function that uses the extracted features as input variables to predict the hemodynamic indicator. Alternatively, the analysis algorithm could be a trained machine learning algorithm that has been trained on a dataset of previous patient data to predict the patient's hemodynamic response based on the extracted features.
In some embodiments, the analysis algorithm may be configured to receive the first set of parameters, generate a first hemodynamic response indicator, and to, separately, receive the second set of parameters and generate a second hemodynamic response indicator. A change in patient fluid responsiveness might then be determined based on comparing the first and second hemodynamic response indicators, for example by subtracting one from the other.

In some embodiments, the analysis algorithm may comprise a classifier algorithm. The hemodynamic response indicator output from the analysis algorithm may be a classification of patient hemodynamic responsiveness into one of a pre-defined set of possible classifications.

Other methods for analyzing waveform features that do not involve classification algorithms include statistical analysis, signal processing techniques, and mathematical modeling. Statistical analysis may involve calculating descriptive statistics, such as mean, median, variance, and correlation coefficients, to summarize the characteristics of the waveform data. Signal processing techniques can include filtering, Fourier analysis, and wavelet transforms to extract information from the signals. Mathematical modeling might involve fitting the waveform data to a mathematical equation or model to understand the underlying physiological processes.

In some embodiments, the analysis algorithm may comprise a machine learning algorithm. For example, in some embodiments, the analysis algorithm may comprise an artificial neural network. In some embodiments, the analysis algorithm may comprise a machine learning classifier algorithm. Alternatively, instead of a machine learning algorithm, a more classical type of algorithm might be used, such as a transfer function, e.g. a multi-parametric regression function.

In some embodiments, the first and second sets of parameters may further include parameters which are computed based on information relating to arterial diameter as a function of time before and after the clinical intervention event.

The method may comprise deriving from the first ultrasound data a first arterial diameter signal representative of a diameter of the blood vessel over said time window contained in the first epoch. The method may comprise deriving from the second ultrasound data a second arterial diameter signal representative of a diameter of the blood vessel over said time window contained in the second epoch. The determining the first set of parameters may further comprise extracting from the first arterial diameter signal one or more pre-defined arterial diameter waveform features. The determining the second set of parameters may further comprise extracting from the second arterial diameter signal the one or more pre-defined arterial diameter waveform features.

In the context of the present disclosure, "arterial diameter signals" may refer to signals that represent or are indicative of the diameter of the blood vessel, or a measure proportional thereto, at the measurement location over a time window contained in the first and second epochs. The measurement location is the location at which the first and second ultrasound data is acquired.

The deriving of the first arterial diameter signal may mean deriving from the first ultrasound data a first arterial diameter signal representative of a diameter of the blood vessel, or a measure proportional thereto, at the measurement location over said time window contained in the first epoch. The measurement location is the location at which the first ultrasound data is acquired.

The deriving of the second arterial diameter signal may mean deriving from the second ultrasound data a second arterial diameter signal representative of a diameter of the blood vessel, or a measure proportional thereto, at the measurement location over said time window contained in the second epoch. The measurement location is the location at which the second ultrasound data is acquired.

With regards to the clinical intervention event, the clinical intervention event may be one or more of: end-expiratory occlusion pressure, inspiratory hold positive end-expiratory pressure 10cm H2O, inspiratory hold positive end-expiratory pressure 15cm H2O, a passive leg raise, and intra-arterial administration of a bolus of liquid to the subject.

However, these are just examples of clinical intervention events. More generally, the same principles can be used to assess hemodynamic response or status following any type of clinical event or intervention, for example a surgical procedure, a treatment, a therapy or simply a patient interaction. The waveform features extracted from the ensemble average blood velocity waveform and/or the other signals will provide a generally applicable insight into changes in hemodynamic status following clinical events.

In some embodiments, each of the first ultrasound data and second ultrasound data comprise both B-mode ultrasound data and pulsed wave Doppler ultrasound data. For example, each of the first and second ultrasound data may be received from an ultrasound system operating in duplex mode, the duplex mode comprising B-mode and pulsed wave Doppler mode.

In some embodiments, the blood velocity-time signal may be derived using the pulsed wave Doppler data. In embodiments in which an arterial dimeter signal is additionally acquired, the arterial diameter signal may be derived using the B-mode data.

In some embodiments, the first and second sets of parameters may additionally include features derived from arterial blood pressure information for the patient, for example from a blood pressure signal for the patient.

Thus, according to one or more embodiments, the method may further comprise obtaining first and second blood pressure measurement information for the subject corresponding to the first and second epochs and wherein the analysis algorithm is further configured to receive the first and second blood pressure measurement information as inputs.

In some embodiments, the obtaining the first and second blood pressure measurement information comprises obtaining first and second blood pressure signals for the subject indicative of a blood pressure waveform for the subject over the time window contained in the first and second epochs respectively. For example, these might be obtained using an invasive arterial line or via an indirect measurement method.

In some embodiments, the method comprises computing a first ensemble average blood pressure waveform from a plurality of blood pressure waveforms present in the first blood pressure signal. Each respective one of the plurality of blood pressure waveforms may correspond to a respective single heartbeat. In other words, each of the plurality of blood pressure waveforms may represent arterial blood pressure as a function of time over the course of a respective single heartbeat. The method may comprise further computing a second ensemble average blood pressure waveform from a plurality of blood pressure waveforms present in the second arterial pressure signal. The first and second blood measurement information may be derived from the first and second ensemble average blood pressure waveforms.

In some embodiments, the determining the first set of parameters comprises extracting from the first ensemble average blood pressure waveform one or more pre-defined blood pressure waveform features. The determining the second set of parameters may comprise computing from the second ensemble average blood pressure waveform the same one or more pre-defined blood pressure waveform features. The blood pressure waveform features extracted from the first and second ensemble average blood pressure waveforms in this case provide the blood pressure measurement information.

In some embodiments, the method comprises deriving from the first ultrasound data a first arterial diameter signal representative of a diameter of the blood vessel over said time window contained in the first epoch, and computing a first ensemble average arterial diameter waveform from a plurality of arterial diameter waveforms present in the first arterial diameter signal. In some embodiments, the method comprises deriving from the second ultrasound data a second arterial diameter signal representative of a diameter of the blood vessel over said time window contained in the second epoch and computing a second ensemble average arterial diameter waveform from a plurality of arterial diameter waveforms present in the second arterial diameter signal. In each case, each respective one of the plurality of arterial diameter waveforms may correspond to a respective single heartbeat. In other words, each of the plurality of arterial diameter waveforms may represent arterial diameter as a function of time over the course of a respective single heartbeat. In some embodiments, the computing the first set of parameters comprises computing from the first ensemble average arterial diameter waveform one or more pre-defined arterial diameter waveform features and computing from the second ensemble average diameter waveform the same one or more pre-defined arterial diameter waveform features.

In other words, the first set of parameters may in some embodiments include the one or more pre-defined arterial diameter waveform features extracted from the first ensemble average diameter waveform and the second set of parameters may include the one or more pre-defined arterial diameter waveform features extracted from the second ensemble average diameter waveform.

In some embodiments, the method may comprise deriving from the first ultrasound data a first arterial diameter signal representative of a diameter of the blood vessel over said time window contained in the first epoch, and computing a first ensemble average arterial diameter waveform from a plurality of arterial diameter waveforms present in the first arterial diameter signal. The method may comprise deriving from the second ultrasound data a second arterial diameter signal representative of a diameter of the blood vessel over said time window contained in the second epoch and computing a second ensemble average arterial diameter waveform from a plurality of arterial diameter waveforms present in the second arterial diameter signal. In each case, each respective one of the plurality of arterial diameter waveforms may correspond to a respective single heartbeat. In other words, each of the plurality of arterial diameter waveforms may represent arterial diameter as a function of time over the course of a respective single heartbeat. The computing the first set of parameters may comprise computing from the first ensemble average arterial diameter waveform one or more pre-defined arterial diameter waveform features. The computing the first set of parameters may comprise computing from the second ensemble average arterial diameter waveform the same one or more pre-defined arterial diameter waveform features.

In other words, the first set of parameters may in this case include the one or more pre-defined arterial diameter parameters extracted from the first ensemble average arterial diameter waveform and the second set of parameters includes the one or more pre-defined arterial diameter parameters extracted from the second ensemble average arterial diameter waveform.

In some embodiments, each of the first and second set of parameters comprise a same group of waveform features which include at least one of: skewness of the ensemble average velocity waveform, kurtosis of the ensemble average velocity waveform, Full Width Half Maximum (FWHM) of the ensemble average velocity waveform, and an acceleration of the ensemble average velocity waveform indicative of a steepness of a leading edge of the ensemble average velocity waveform.

"Skewness" refers to a measure of the asymmetry of the probability distribution of a real-valued random variable about its mean. "Kurtosis" refers to a measure of the "tailedness" of the probability distribution of a real-valued random variable. In mathematics, skewness is sometimes understood as the third standardized moment of the probability distribution, and kurtosis may be understood as the fourth standardized moment of the probability distribution. "Full Width Half Maximum (FWHM)" is a measure of the width of a pulse at half of its maximum intensity.

In some embodiments, each of the first and second sets of parameters include a value for an area under a pressure-volume loop, the pressure-volume loop computed using the ensemble average blood velocity waveform, the ensemble average blood pressure waveform and the arterial diameter signal.

In some embodiments, each of the first and second set of parameters include a value for a steepness of the rising edge (or upslope) of a pressure-volume loop, the pressure volume loop computed using the ensemble average blood velocity waveform, the ensemble average blood pressure waveform and the arterial diameter signal.

In some embodiments, each of the first and second set of parameters may include values for one or more waveform features extracted from the velocity-time signal, for example any one or more of: peak systolic velocity, end diastolic velocity, area under the velocity-time signal per heartbeat, mean velocity, mean velocity per heartbeat, mean square velocity, and inter-decile range of the velocity-time signal.

The area under the velocity-time signal per heartbeat can be calculated as the integral of the velocity-time signal over the relevant time interval across which the signal spans, divided by the number of blood velocity waveforms (i.e. the number of heartbeats) which are present in that time interval.

The mean velocity per heartbeat can be calculated as the mean of the velocity-time signal samples over the relevant time interval across which the signal spans, divided by the number of blood velocity waveforms (i.e. the number of heartbeats) which are present in that time interval.

In some embodiments, each of the first and second set of parameters may include values for each of one or more features which are computed from a combination of the velocity-time signal and the arterial diameter signal, from a combination of the velocity-time signal and the diameter signal, and/or from a combination of the arterial diameter signal and the blood pressure signal.

For example, in some embodiments, each of the first and second set of parameters may include a value for a square of the difference between squared values of peak systolic velocity (PSV) and end diastolic velocity (EDV) divided by squared value of diameter squared. This is referred to by the label 'deltaVelByDia' in the detailed description section of this document.

By way of further example, in some embodiments, each of the first and second set of parameters may include a value for mean blood pressure squared divided by a value of peak systolic velocity (PSV). The value for the mean blood pressure squared can be derived from the arterial blood pressure signal and the value for the PSV can be derived from the velocity-time signal. This feature is referred to by the label 'ABPmSquaredByPSV' in the detailed description.

By way of further example, in some embodiments, each of the first and second set of parameters may include a value for peak arterial blood pressure squared divided by mean arterial diameter. This feature is referred to by the label 'ABPpSquaredbyMeanDia' in the detailed description.

By way of further example, in some embodiments, each of the first and second set of parameters may include a value for the square of mean flow rate over a time interval. The mean flow rate can be computed from the velocity-time signal in combination with the arterial diameter signal.

In accordance with another aspect of the invention, a computer program product is provided comprising computer code configured to cause a processor to perform the method in accordance with any embodiment or example described in the disclosure or in accordance with any claim.

In accordance with another aspect of the invention, a processing device is provided comprising one or more processors configured to perform the method in accordance with any embodiment or example described in the disclosure or in accordance with any claim.

In accordance with another aspect of the invention, a system is provided comprising the processing device as described above and an ultrasound acquisition apparatus for acquiring the first and/or second ultrasound data. The ultrasound system may be operable in duplex mode for generating B-mode and pulsed wave Doppler ultrasound data. In some embodiments, the ultrasound acquisition apparatus comprises a wearable ultrasound transducer unit. The wearable ultrasound transducer unit may comprise a wearable component which comprises an integrated ultrasound transducer array.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example processing device and system in accordance with one or more embodiments of the invention;

Fig. 3 illustrates example waveform features which may be extracted from an ensemble average velocity waveform in accordance with one or more embodiments;

Fig. 4 shows an example arterial pressure signal and an example velocity-time signal and indicates signal landmarks which might be used to extract a plurality of pulses from each signal for computing respective ensemble average waveforms;

Fig. 5 illustrates a distribution of kurtosis values of a plurality of ensemble average velocity waveforms computed prior to a clinical intervention event and after the clinical intervention event; and

Fig. 6 shows the distribution of the differences between the kurtosis values of Fig. 5.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for evaluating changes in patient hemodynamic status, e.g. hemodynamic responsiveness, between before and after clinical intervention events. Waveform features extracted from an ensemble average blood velocity waveform for the patient are fed as input to an analysis algorithm, for example comprising a classifier, to generate an output indicative of one or more hemodynamic characteristics, for example indicative of hemodynamic responsiveness. This can be used to evaluate changes in hemodynamic responsiveness before and after a clinical intervention event.

This could for example be used for the purpose of alerting clinicians as to unstable /unresponsive hemodynamic characteristics.

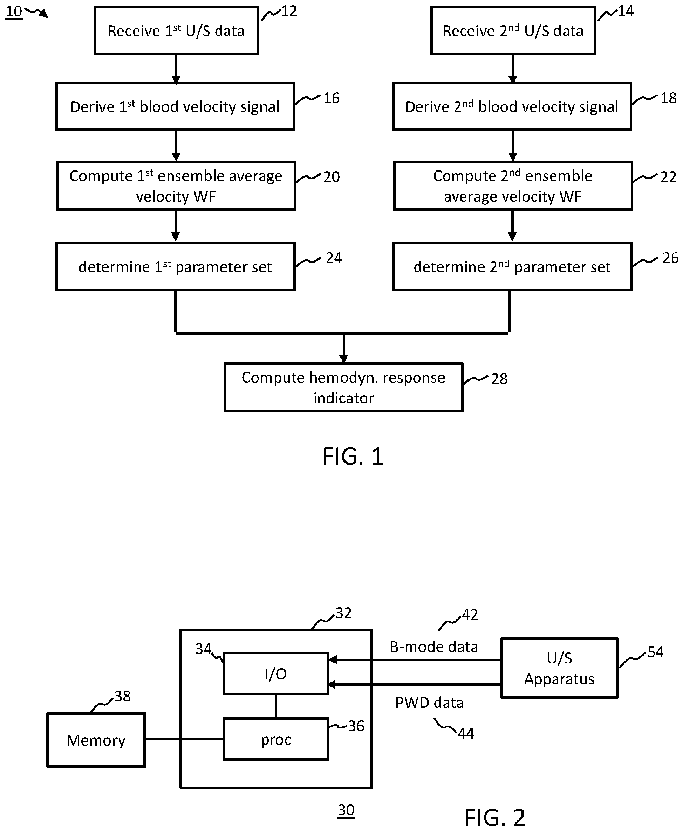

Fig. 1 outlines in block diagram form steps of an example method according to one or more embodiments. The steps will be recited in summary, before being explained further in the form of example embodiments.

The method 10 comprises receiving 12 first ultrasound data of a blood vessel of a subject. The first ultrasound data is acquired over a first epoch, which occurs prior to a clinical intervention event or patient interaction event. The method 10 further comprises receiving 14 second ultrasound data of the same blood vessel of the subject. The second ultrasound data is acquired over a second epoch, which occurs following the clinical intervention event or patient interaction event.

Using the first ultrasound data, a first blood velocity-time signal is derived 16. This signal is representative of blood velocity over a time window contained in the first epoch. A first ensemble average blood velocity waveform is then computed 20 from a plurality of blood velocity waveforms present in the first blood velocity-time signal 16. Similarly, from the second ultrasound data, a second blood velocity-time signal is derived 18. This signal is representative of blood velocity over a time window contained in the second epoch. A second ensemble average blood velocity waveform 22 is then computed from a plurality of blood velocity waveforms present in the second blood velocity-time signal 18. These ensemble average blood velocity waveforms may provide a representative measure of the blood velocity within the blood vessel over the time windows contained in the first and second epochs.

The method 10 further comprises determining 24 a first set of parameters. This comprises extracting from the first ensemble average blood velocity waveform one or more pre-defined blood velocity waveform features. A second set of parameters is also determined 26. This comprises extracting from the second ensemble average blood velocity waveform the one or more pre-defined blood velocity waveform features.

These waveform features may provide information about the characteristics of the blood flow within the blood vessel, such as its velocity, acceleration, and/or deceleration.

The method 10 also comprises retrieving and applying a pre-defined analysis algorithm. The analysis algorithm is configured to receive the first and second sets of parameters as inputs and to generate 28 a hemodynamic response indicator as an output. The hemodynamic response indicator is for example indicative of characteristics of a patient's hemodynamic responsiveness to the clinical intervention event.

In some embodiments, the method 10 may further comprise deriving from the first ultrasound data a first arterial diameter signal representative of a diameter of the blood vessel over the time window contained in the first epoch. Similarly, from the second ultrasound data, a second arterial diameter signal may be derived, representative of a diameter of the blood vessel over the time window contained in the second epoch. The determining of the first set of parameters may further comprise extracting from the first arterial diameter signal one or more pre-defined arterial diameter waveform features. Similarly, the determining of the second set of parameters may further comprise extracting from the second arterial diameter signal the one or more pre-defined arterial diameter waveform features.

Fig. 2 presents a schematic representation of an example processing device 32 configured to execute the method 10 in accordance with one or more embodiments of the present disclosure. The processing device 32 is shown in the context of a system 30 which comprises the processing device. The processing device 32 alone represents an aspect of the invention. The system 30 is another aspect of the invention. The provided system 30 may not comprise all the illustrated hardware components; it may comprise a subset of them.

The processing device 32 comprises one or more processors 36 configured to perform the method 10 in accordance with the steps outlined above. In the illustrated example, the processing device 32 further comprises an input/output interface 34 or communication interface. In the illustrated example of Fig. 2, the system 30 further comprises an ultrasound acquisition apparatus 54 for acquiring ultrasound data, for example including B-mode ultrasound data 42 and Pulse Wave Doppler ultrasound data 44. In some cases, the ultrasound acquisition apparatus 54 may comprise a wearable ultrasound transducer unit, such as patch having an ultrasound transducer array integrated therein. The system 30 may further comprise a memory 38 for storing computer program code (i.e., computer-executable code) which is configured for causing the one or more processors 36 of the processing unit 32 to perform the method 10 as outlined above.

In some embodiments, the first and second ultrasound data may comprise both B-mode ultrasound data 42 and pulsed wave Doppler data 44. The B-mode ultrasound data 42 may provide information about the structure of the blood vessel, such as its diameter, while the pulsed wave Doppler data 44 may provide information about the velocity of blood flow within the blood vessel.

As mentioned above, the method 10 may involve deriving 16 a first blood velocity-time signal from the first ultrasound data 12, and deriving a 18 a second blood velocity-time signal from the second ultrasound data 14. These signals may be representative of blood velocity over a time window contained in the first and second epochs, respectively. The blood velocity-time signals may be derived using the pulsed wave Doppler data 44, which may provide a measure of the speed and direction of blood flow within the blood vessel.

As mentioned above, in some embodiments, the method 10 may further comprise deriving a first arterial diameter signal and a second arterial diameter signal from the first and second ultrasound data respectively. These arterial diameter signals may be representative of the diameter of the blood vessel over the (same) time windows contained in the first and second epochs, respectively. The arterial diameter signals may in some examples be derived using the B-mode ultrasound data 42, which may provide a measure of the size and shape of the blood vessel.

The method 10 may involve computing a first ensemble average blood velocity waveform 20 from the first blood velocity-time signal 16, and a second ensemble average blood velocity waveform 22 from the second blood velocity-time signal 18. The ensemble average blood velocity waveforms may be computed from a plurality of blood velocity waveforms present in the blood velocity-time signals.

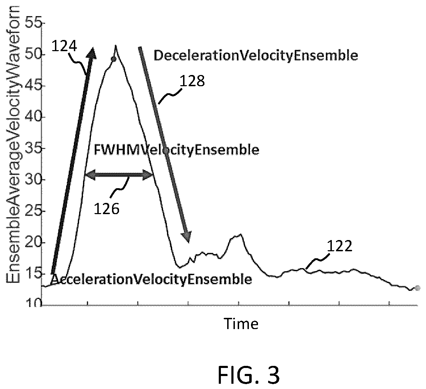

Fig. 3 provides a graphical representation of an example ensemble average blood velocity waveform 122. The ensemble average blood velocity waveform 122 is derived from the first and second blood velocity-time signals which are representative of blood velocity over a time window contained in the first and second epochs, respectively. The ensemble average blood velocity waveform 122 is computed from a plurality of blood velocity waveforms present in the blood velocity-time signals.

The extracted features from the ensemble average waveform, such as the deceleration 124, the full width at half maximum (FWHM) 126, and the acceleration 128, provide information about the hemodynamic status of the patient.

Values for one or more of these features may be computed to form values for each of the first and second sets of parameters 24, 26 which are then input into the pre-defined analysis algorithm to compute 28 the hemodynamic response indicator.

For each of the first and second blood velocity-time signal, the method may comprise extracting a plurality of blood velocity waveforms. This process may comprise detecting one or more landmark or fiducial features in the respective signal and then extracting the individual pulse waveforms based thereon.

The computing an ensemble average blood velocity waveform may comprise aligning the plurality of extracted pulse waveforms, for example based on aligning common fiducial points identified in each of the plurality of waveforms, for example aligning peaks and/or troughs identified in the plurality of pulse waveforms.

The ensemble average velocity waveform 122 may be characterized by several waveform features. These waveform features may be extracted or derived from the ensemble average blood velocity waveform. One waveform feature is an acceleration 124 of the ensemble average blood velocity waveform indicative of a steepness of a leading edge of the ensemble average blood velocity waveform. Another feature is a deceleration 128 of the ensemble average blood velocity waveform indicative of a steepness of a trailing edge of the ensemble average blood velocity waveform. Another feature is a full width half maximum (FWHM) 126 of the ensemble average blood velocity waveform representative of the width of the waveform at half of its maximum height.

Other waveform features which might be extracted from the ensemble average blood velocity waveform include skewness of the ensemble average blood velocity waveform and kurtosis of the ensemble average blood velocity waveform.

In general, the skewness of a waveform provides a measure of the asymmetry of the probability distribution of a real-valued random variable about its mean. In general, the kurtosis of a waveform provides a measure of the "tailedness" of the probability distribution of a real-valued random variable. The FWHM of the ensemble average blood velocity waveform may provide a measure of the dispersion of the waveform.

These waveform features may be extracted from the ensemble average blood velocity waveforms and may be used in the generation 28 of the hemodynamic response indicator as discussed above. The hemodynamic response indicator 28 may provide an indication of the patient's hemodynamic responsiveness or hemodynamic status more generally.

In some embodiments, the method 10 may further involve obtaining first and second blood pressure measurement information for the subject corresponding to the first and second epochs. This blood pressure measurement information may provide additional insights into the patient's hemodynamic status following the clinical intervention event.

The first and second blood pressure measurement information may be obtained from first and second blood pressure signals spanning respective time windows contained in the first and second epochs respectively.

In some cases, the method 10 may involve computing a first ensemble average blood pressure waveform from a plurality of blood pressure waveforms present in the first blood pressure signal, and further computing a second ensemble average blood pressure waveform from a plurality of blood pressure waveforms present in the second blood pressure signal. The ensemble average blood pressure waveforms may provide a representative measure of the arterial blood pressure within the blood vessel over said time windows contained in the first and second epochs.

The first and second blood pressure measurement information may be derived from the first and second ensemble average blood pressure waveforms. This derivation may involve extracting one or more pre-defined blood pressure waveform features from the ensemble average blood pressure waveforms. These blood pressure waveform features may provide information about the characteristics of the arterial blood pressure within the blood vessel, such as for example its maximum and minimum values, and/or its rate of change with respect to time.

In some embodiments, the determining of the first set of parameters 24 may comprise extracting from the first ensemble average blood pressure waveform one or more pre-defined blood pressure waveform features. Similarly, the determining of the second set of parameters 26 may comprise extracting from the second ensemble average blood pressure waveform the same one or more pre-defined blood pressure waveform features. These blood pressure waveform features may provide additional information about the patient's hemodynamic status following the clinical intervention event. They may in some embodiments be used in conjunction with the blood velocity waveform features mentioned previously to generate the hemodynamic response indicator 28.

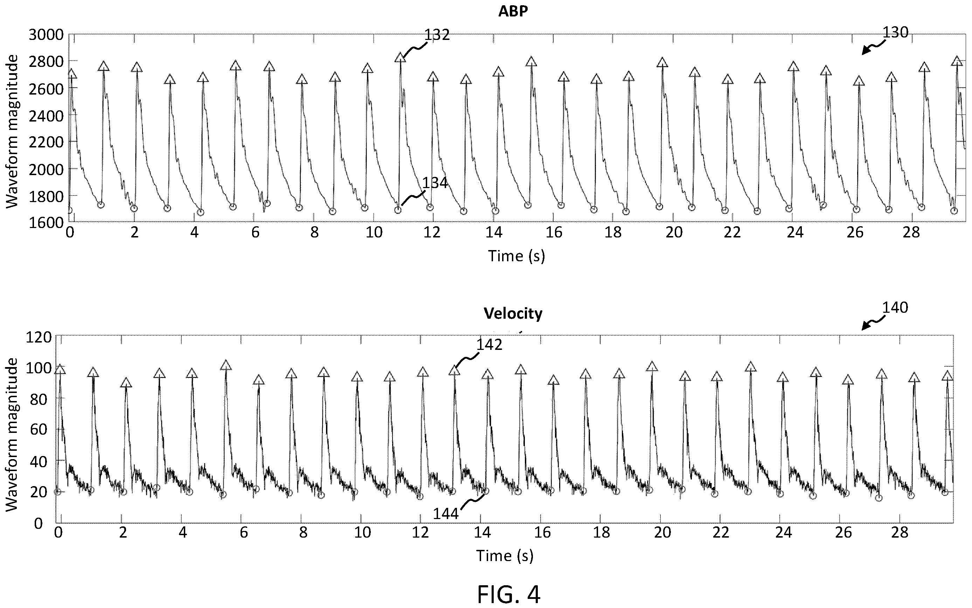

Fig. 4 shows a graphical representation of an arterial blood pressure (ABP) signal 130 and a blood velocity-time signal 140.

To derive an ensemble average blood velocity waveform, ensemble average blood pressure waveform, or ensemble average diameter waveform, the method involves extracting multiple waveforms from the original relevant signal, for example each corresponding to a single respective pulse or heartbeat. This process begins by detecting fiducial points within the relevant signal (velocity-time signal, blood pressure signal, diameter signal) which correspond to specific, identifiable points in the cardiac cycle.

As shown in Fig. 4, fiducial detection involves identifying landmarks. For the ABP signal 130, this may comprise detecting maxima points, marked as ABP maxima 132 in Fig. 4, and minimum points, marked as ABP minima 134 in Fig. 4. For the blood velocity signal 140, this may comprise detecting blood velocity maxima 142 and blood velocity minima 144. These points may serve as reference markers for aligning the individual waveforms.

Once the fiducial points are detected, the individual waveforms may be extracted and aligned based on these points. This alignment ensures that the waveforms are synchronized in time, allowing for accurate averaging. The aligned waveforms are then averaged together to create an ensemble average waveform. This ensemble average waveform represents a composite of the individual waveforms and provides a more stable and representative measure of the underlying hemodynamic signal. The ensemble average waveform for example corresponds to a single representative pulse or heartbeat or heart cycle.

The ensemble average waveform, whether it be for velocity, blood pressure, or diameter, is characterized by features that are less susceptible to the variability and noise present in individual waveforms. By averaging across multiple cardiac cycles, the method enhances the signal-to-noise ratio, allowing for more reliable extraction of waveform features.

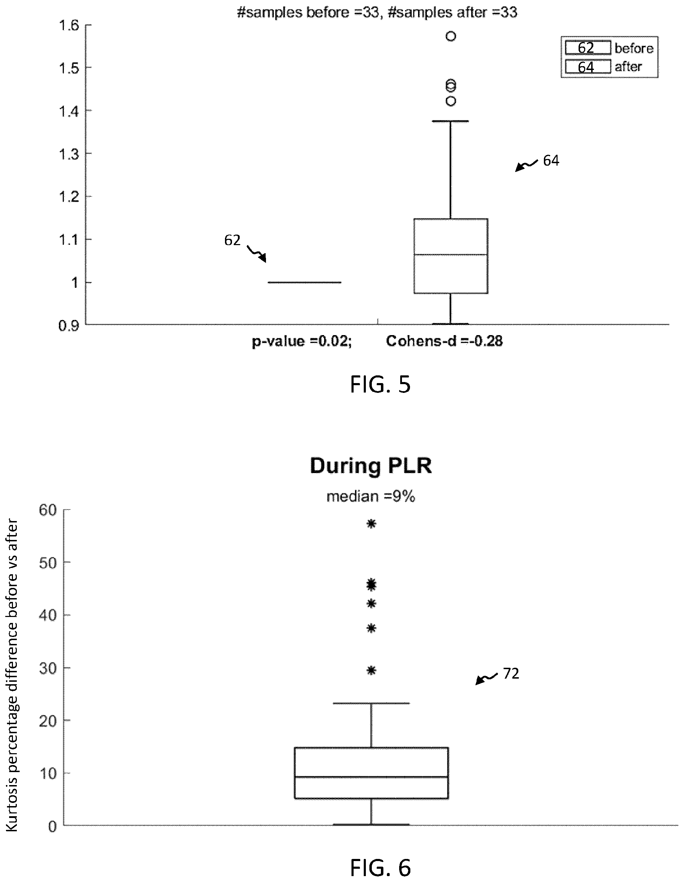

With reference to Fig. 5 and Fig. 6, these figures provide graphical representations of distribution of values of kurtosis computed for a plurality of ensemble average blood velocity waveforms derived for a patient before and after a passive leg raise (PLR) event.

In particular, Fig. 5 is a box plot comparison of ensemble average velocity kurtosis values before and after the PLR event.

The box in each plot represents the middle 50% of the data. The bottom of the box is the first quartile (Q1, 25th percentile), and the top is the third quartile (Q3, 75th percentile). The distance between them is the interquartile range (IQR).

The horizontal line inside the box is the median, which divides the data into two halves.

The whiskers extending from the top and bottom of the box represent the range of the data, extending to the minimum and maximum values within 1.5 IQRs from the quartiles. Data points outside this range are considered outliers.

The box plot on the left represents the distribution of data before the clinical event. The median value is around 1.2. The box plot on the right represents the data after the clinical event. The median value is slightly lower than before, approximately 1.1.

There is a p-value of 0.02. This indicates that there is a statistically significant difference between the data before and after the event. A p-value below 0.05 generally suggests significant differences.

There is a Cohen's d value of -0.28: This value is a measure of the effect size, indicating the magnitude of the difference between the two groups.

In summary, the graph shows that there's a statistically significant reduction in the median value of the measured parameter after the clinical event. This change is consistent across the distribution as seen in the shift of the box and the adjustment in the spread of the data (including outliers and range).

Fig. 6 is a box plot showing the distribution of absolute percentage differences in kurtosis values between the 'before' and 'after' samples represented in Fig. 5.

The box shows the interquartile range (IQR), where the bottom of the box represents the 25th percentile, and the top of the box represents the 75th percentile.

The line within the box shows the median of the data, which is 9%.

The lines extending from the box show the range of the data close to the box, within 1.5 times the IQR from the quartiles. In this graph, the lower whisker extends to the bottom 0% line, and the upper whisker goes up to about 10%.

The data points above the upper whisker represent outliers, which are data points that fall outside the typical range defined by the whiskers. These outliers show higher absolute percentage differences, up to 60%.

The median absolute percentage difference is 9%, indicating that the typical difference between kurtosis values of the 'before' and 'after' samples centers around this value.

The spread from the minimum at 0% to the top of the upper whisker around 10% indicates that most data points have a difference of 10% or less. However, there are several outliers which show much larger differences, significantly higher than the typical range.

The data indicates that most of the samples demonstrate a statistically significant change after the event of around median 10%, with some samples demonstrating a much larger difference (up to 60%).

Although in the example of Fig. 5 and Fig. 6 the clinical intervention was a PLR event, the clinical intervention event in the method 10 may be any other intervention event, such as a treatment, a surgical procedure, an end-expiratory occlusion pressure, inspiratory hold positive end-expiratory pressure 10cm H2O, inspiratory hold positive end-expiratory pressure 15cm H2O, and intra-arterial administration of a bolus of liquid to the subject.

In some embodiments, the method 10 may involve analyzing the extracted waveform features for an ensemble average velocity waveform computed before and after the PLR event to determine the patient's hemodynamic response to the event. This may involve computing the absolute percentage difference in the kurtosis values, determining the median percentage change, and assessing the spread of the data points. The resulting hemodynamic response indicator 28 may provide an indication of the patient's hemodynamic responsiveness to the PLR event.

With regards to the waveform features which may be extracted from the ensemble average velocity waveform, and/or the other waveforms and/or signals which have been discussed above, the inventors have identified and tested a comprehensive set of waveform features which have been found to be good predictors or correlates of changes in hemodynamic status (e.g. responsiveness) before and after a clinical event. A list of these features will now be presented, along with the formula for their calculation.

There will first be presented a list of example waveform features which may be extracted from the ensemble average velocity waveform:

*Table 1*

| Feature | Description | Formula | Unit |
| --- | --- | --- | --- |
| AccelerationVelocityEnsemble | Acceleration of ensemble average velocity WF from diastole to PSV | $\dfrac{PSV - EDV}{PSV(t) - EDV(t)}$ | cm/s^2 |
| FWHMVelocityEnsemble | Time (full width) at half the max value of ensemble average velocity WF | - | s |

(continued)

| Feature | Description | Formula | Unit |
|---|---|---|---|
| RelFWHMVelocityEnsemble | Relative time of the FWHM over the cardiac cycle | proportion of the total pulse duration that the Full Width at Half Maximum (FWHM) occupies | No unit |
| RelAccelerationTimeVelocityEnsemble | Ratio of time to PSV to cardiac cycle duration | PSV(t)/ cardiac cycle length | No unit |
| FW3QM_To_FWHMVelocityEnsemble | Ratio of full width at 75% of Max to ratio of full width at 50% of max | | No unit |
| DecelerationVelocityEnsemble | Deceleration of velocity as measured from to systolic peak to subsequent foot | $$\frac{PSV - EDV}{EDV(t) - PSV(t)}$$ | cm/s^2 |
| PSV = velocity at peak systolic velocity<br>EDV = velocity at end diastolic velocity<br>PSV(t) = time at peak systolic velocity<br>EDV(t) = time at end diastolic velocity<br>WF = "waveform" | | | |

As discussed above, each of the first and second sets of parameters may comprise one or more waveform features extracted from a blood pressure signal and/or from an ensemble average blood pressure waveform. There will now be outlined a set of example waveform features which may be extracted from a blood pressure signal and/or from an ensemble average blood pressure waveform, any one or more of which may be utilized as part of the first and second sets of parameters.

*Table 2*

| Feature | Description | Formula | Unit |
|---|---|---|---|
| AccelerationABPEnsemble | Acceleration of ensemble average ABP pulse WF from diastole to systole | $$\frac{PSP - EDP}{PSP(t) - EDP(t)}$$ | mmHg/s |
| FWHMABPEnsemble | Full Width at half the max value of systolic pressure ensemble average ABP pulse WF | - | s |
| RelFWHMABPEnsemble | Relative time of the FWHM over the total pulse width of ensemble average ABP pulse WF | proportion of the total pulse duration that the Full Width at Half Maximum (FWHM) occupies | No unit |
| RelAccelerationTimeABPEnsemble | Ratio between time to systole (from diastole) to cardiac cycle duration of ensemble average ABP pulse WF | PSP(t) / total pulse width | No unit |
| FW3QM_To_FWHMABPEnsemble | Ratio of full width at 75% of Max to ratio of full width at 50% of max of ensemble average ABP pulse WF | | No unit |
| DecelerationABPEnsemble | Deceleration of ensemble average ABP pulse WF as measured from systole to subsequent diastole | $$\frac{PSP - PDP}{PDP(t) - PSP(t)}$$ | mmHg/s |

(continued)

| Feature | Description | Formula | Unit |
|---|---|---|---|
| SignalStabilityIndexABP | Signal stability index (SSI) of standardized ABP waveform | e.g. based on choosing the peak of the summated kernel density estimator | No unit |
| SignalInstabilityIndexABP | Signal instability index of standardized ABP waveform | Inverse of SSI | No unit |

ABP = arterial blood pressure
PSP = peak systolic pressure (value of the pressure signal at peak systole)
EDP = end diastolic pressure (value of the pressure signal and end diastole)
PSP(t) = time at peak systolic pressure
EDP(t) = time at end diastolic pressure
"WF" = waveform

As mentioned above, in some embodiments, each of the first and second sets of parameters may include one or more waveform features extracted from an arterial diameter signal computed from the ultrasound data. There will now be outlined a list of example waveform features which may be extracted from an arterial diameter signal, any one or more of which may be included in the first and/or second sets of parameters in accordance with one or more embodiments.

*Table 3*

| Feature | Description | Formula | Unit |
|---|---|---|---|
| MeanDiameter | Average Diameter | Mean[d(i)] | cm |
| MeanCrossSectionalArea | Cross sectional area of diameter (average) | $$\frac{\pi * (Mean[d(i)])^2}{4}$$ | cm² |
| IDRDiameter | Interdecile range of diameter signal | Percentile[d(i), 0.9] - Percentile[d(i), 0.1] | cm |
| SkewnessDiameter | skewness of diameter signal | - | No unit |
| KurtosisDiameter | kurtosis of diameter signal | - | No unit |
| SignalStabilityIndexDiameter | Signal stability index (SSI) of diameter signal | e.g. based on choosing the peak of the summated kernel density estimator | No unit |
| SignalInstabilityIndexDiameter | Signal instability index (SII) of diameter signal | Inverse of Signal Stability Index (SSI) | No unit |

d(i) = arterial diameter signal as a function of sample number, i, where samples are acquired at time intervals over the course of each heart cycle
Mean[d(i)] = mean blood vessel diameter over a single waveform for a single heart cycle
Percentile[d(i), 0.9] = 90th percentile of diameter signal, d(i)
Percentile[d(i), 0.1] = 10th percentile of diameter signal, d(i)

Signal Stability Index (SSI) measures the degree to which a signal remains consistent over time or across different samples. Higher values typically indicate a more stable or consistent signal.

In a general case, a measure of SSI for a signal can be computed by segmenting a signal into multiple time windows and analyzing the variance or other statistical properties within and across these windows. This approach is applicable in embodiments of the present invention. Additionally or alternatively, computation of SSI may be based on choosing the peak of the summated kernel density estimator.

Signal Instability Index (SII) measures the degree to which a signal changes, fluctuates, or deviates from some norm over time. Higher values indicate more variability or instability. This can be understood as the inverse of SSI.

As mentioned above, in some embodiments, at least one of the first and second sets of parameters may include one or more waveform features extracted from a velocity-time signal for a given pulse (i.e. a non ensemble-averaged velocity pulse). An example set of waveform features which might be extracted from a velocity-time signal will now be outlined, any one or more of which may be included among the first and/or second sets of parameters.

*Table 4*

| Feature | Description | Formula | Unit |
|---|---|---|---|
| Peak Systolic Velocity | Peak Systolic velocity | Max[v(i)] for i=[0, signal window] | cm/s |
| MeanVelocity | Mean of velocity-time signal | Mean[v(i)] | cm/s |
| SystolicFootVelocity | Systolic foot (or trough) | Min[v(i)] for i=[0... signal window] | cm/s |
| IDRVelocity | Interdecile range of velocity-time signal | Percentile [v(i), 0.9] -Percentile [v(i), 0.1] | cm/s |
| VtiPerBeat | Area under the velocity-time signal (VTI) per heart beat | $\left( \sum_{i=1}^{n} v(i) \right)$/ Sample Freq/ No of hearbeats | cm |
| MeanVelocityPerBeat | Mean velocity divided by the number of heartbeats in the epoch | Mean[v(i)]/ No of heartbeats | cm/s |
| Signal Stability Index Velocity | Signal stability index of standardized velocity signal | | No unit |
| SignalnstabilityIndexVelocity | Signal instability index standardized velocity signal | Inverse of SSI | No unit |
| MeanHR | Average heart rate in the epoch | no of heart beats / epoch duration | bpm |
| SqrtMeanHR | sqrt of mean HR | sqrt(mean HR) | $bpm^{1/2}$ |
| MeanVelocitySquare | Mean of velocity squared | Mean $[(v(i))]^2$ | $cm^2/s^2$ |
| SqrtPeakSystolicVelocity | Square root of PSV | sqrt(PSV) | $(cm/s)^{1/2}$ |
| SkewnessVelocity | skewness of velocity waveform | - | No unit |
| KurtosisVelocity | kurtosis of velocity waveform | - | No unit |
| PulsatilityIndexVelocity | Peak - systolic amplitude of velocity waveform divided by mean value of waveform | (PSV - EDV)/ Mean velocity | No unit |
| SDNN | The standard deviation of the NN intervals within a time-window. Note : NN interval is 'normal' RR intervals | std(RRi) | ms |
| RMSSD | The square root of the mean of the squares of successive differences between adjacent NN-intervals within a time-window. | rms (RRi) | ms |

v(i) = value of blood velocity as a function of sample number, i, where samples are acquired at time intervals
PSV = velocity at peak systolic velocity
EDV = velocity at end diastolic velocity
PSV(t) = time at peak systolic velocity
EDV(t) = time at end diastolic velocity
Percentile[d(i), 0.9] = 90th percentile of diameter signal, d(i)
Percentile[d(i), 0.1] = 10th percentile of diameter signal, d(i)
NN interval = the time period between successive normal cardiac R-wave peaks, and can be inferred from the systolic peak landmarks in the velocity-time signal

=

In some embodiments, the method may further comprise deriving a pressure-volume loop using an arterial pressure waveform and a blood volume waveform both spanning the same measurement time interval. A blood volume waveform can be computed from a blood velocity waveform by multiplying each velocity value by a value for arterial cross-sectional area, where arterial cross-sectional area can be estimated from a value derived for arterial diameter. If an arterial diameter signal exists spanning the same measurement time interval as for the pressure and velocity signals, then the time-varying diameter signal may be converted into a time-varying cross-sectional area signal, and then the cross-sectional area signal may be multiplied by the blood velocity signal to obtain a blood volume signal. As will be well known to the skilled person, a pressure-volume loop (or PV loop) can be used to approximate work done by the heart. In accordance with some embodiments of the invention, the first set of parameters may include one or more waveform features extracted from a PV loop computed for a cardiac cycle. The PV loop may optionally be computed using an ensemble average velocity waveform and ensemble average pressure waveform. Two examples of such waveform features are outlined below:

*Table 5*

| Feature | Description | Formula | Unit |
|---|---|---|---|
| EnsemblePVLoopWorkDoneFeatur e | Area under the ensemble average PV loop as proxy for work done by the heart. | - | J |
| EnsemblePVLoopUpslopeFeature | Slope of PV Loop Upslope | tan [(Max[Vol]-Min [Vol]) / (Max[p] -Min [p])] | cm$^3$/mmHg |

Max[Vol] is the maximum volume value of the PV loop
Min[Vol] is the minimum volume value of the PV loop
Max[p] is the maximum pressure value of the PV loop
Min[p] is the minimum pressure value of the PV loop

In some embodiments, each of the first and second set of parameters may include waveform features computed using a combination of the different waveforms and/or signals discussed above. Some examples will now be outlined below.

*Table 6*

| Feature | Description | Formula | Unit |
|---|---|---|---|
| MeanFlowRate | Mean flow rate wave-form | $\frac{\pi}{4} \, mean(\, d(i)^2 * v(i)\,)$ | cm$^3$/s |
| MeanFlowRatePerBeat | Mean flow rate per beat | $\frac{\pi}{4 * No\ of\ heart\ beats} \, mean(\, d(i)^2 * v(i)\,)$ | cm$^3$/s |
| MeanPeakFlowRate | Flow (cm^3/s) estimated with PSV x Cross section | $\frac{\pi * AvgDia^2}{4} \, mean(PSV)$ | cm$^3$/s |
| MeanFlowRate Squared | Square of mean flow rate | square (MeanFlowRate) | cm$^6$/s$^2$ |
| DeltaVelbyDia | Square of difference between squared values of PSV and EDV divided by squared value of diameter squared | $\frac{(PSV^2 - EDV^2)^2}{Diameter^2}$ | cm$^2$/s$^4$ |
| SqrtPSVByDiaSq | Square root of PSV times Diameter squared | $\frac{sqrt(PSV)}{Diameter^2}$ | 1/(cm*s) |
| ABPmSquaredByPSV | mean blood pressure squared divided by PSV | | (mmHg^2) / (cm/s) |

(continued)

| Feature | Description | Formula | Unit |
|---|---|---|---|
| ABPpSquaredbyMeanDia | pulse pressure squared divided by mean arterial Diameter | | (mmHg) / (cm) |
| v(i) = value of blood velocity as a function of sample number, i, where samples are acquired at time intervals<br>d(i) = arterial diameter signal as a function of sample number, i, where samples are acquired at time intervals over the course of each heart cycle<br>PSV = velocity at peak systolic velocity<br>EDV = velocity at end diastolic velocity<br>PSV(t) = time at peak systolic velocity<br>EDV(t) = time at end diastolic velocity | | | |

The predictive power of the different waveform features to differentiate clinical events has been evaluated by the inventors. This analysis was performed using data collected during four different controlled clinical intervention events which were performed for a cohort of patients during an ultrasound measurement period. The clinical intervention events under study were end-expiratory occlusion pressure (EEOP), inspiratory hold positive end-expiratory pressure (PEEP) 10cm H2O, inspiratory hold PEEP 15cm H2O, and a passive leg raise (PLR).

The analysis encompassed a total of 46 EEOP events, 66 inspiratory hold PEEP 10cm H2O events, 36 inspiratory hold PEEP 15cm H2O events, and 33 PLR events with ultrasound data acquired over a respective first epoch before each event and a second epoch after each event and also an epoch during each event.

For this analysis, the inventors selected an epoch before and another after the event took place. The epoch before the event was selected to be the one just before the next event took place, while the epoch after was selected to be two epochs prior to the subsequent event.

For each waveform feature and for each analyzed clinical intervention event, the data was normalized by the feature value before the event. The absolute percentage difference was calculated as follows:

$$\text{absolute percentage difference} = 100 \times \frac{feat_{after} - feat_{before}}{feat_{before}}$$

where $feat_{after}$ is the waveform feature value after the event and $feat_{before}$ is the waveform feature value before the event.

To quantify waveform feature differences before and after the event, the Cohen's d effect size metric was used. A Cohen's d was calculated assuming paired samples using the Matlab function *meanEffectSize(X, Y, Paired-true, Effect="cohen")*. Negative Cohen's-d values highlight negative changes induced by the event, i.e., the values after the event were lower than those before. It is generally taken to be the case that d values of 0.2 are indicative of a small effect size, a medium effect size is represented by d = 0.5, and d = 0.8 are a result of large effect sizes.

A non-parametric Wilcoxon Signed-rank test was used to test for statistically significant differences in the feature values observed before and after each event. As the skilled person will be aware, this is a non-parametric test used to compare two paired samples to assess whether their population mean ranks differ. The test focuses on the median of the differences between pairs, rather than the mean, and assesses whether the median differs from zero in a symmetric distribution of differences.

An example visual representation of the normalized feature values and absolute percentage difference has been presented in Fig. 5 and Fig. 6, and this has been discussed above.

In Table 7 below are recited a list of the waveform features that exhibited a median absolute percentage difference higher or equal to 5% and a p-value $\leq 0.1$, categorized by the type of input data source necessary for their derivation. Multiple features were found to show significant percentage changes for all the controlled events examined.

For example, simple velocity-only features such as *MeanVelocitySquare* were found to have a significant percentage change during PLR events. More complex velocity-only features such as *Skewness Velocity* were also found to show similar magnitudes of percentage change as *MeanVelocitySquare* during PLR; however, when comparing the Cohen's d values between these features it was observed that the more complex features exhibited a stronger effect (-0.28 vs 0.12), highlighting the additional benefit of these more complex features.

*Table 7*

| Necessary inputs | Feature name | Clinical Intervention Event | Median absolute percentage difference | Cohen's d | p-value |
|---|---|---|---|---|---|
| *Velocity Ensemble + Pressure Ensemble + Diameter* | EnsemblePVLoopWorkDon e | EEOP | 39% | -0.25 | 0.01 |
| | EnsemblePVLoop UpSlope | EEOP | 5% | 0.12 | 0.07 |
| *Velocity Ensemble* | KurtosisVelocity | PLR | 9% | -0.28 | 0.02 |
| | SkewnessVelocity | PLR | 7% | -0.25 | 0.02 |
| | FWHMVelocityEnsemble | PLR | 7% | -0.09 | 0.10 |
| | RelAccelerationTimeVelocit yEnsemble | EEOP | 6% | -0.05 | 0.09 |
| | AccelerationVelocity Ensem- ble | PEEP 15cm $H_2O$ | 6% | 0.06 | 0.08 |
| *Velocity + Diameter* | deltaVelByDia | PLR | 35% | 0.14 | 0.08 |
| | | EEOP | 16% | -0.06 | 0.07 |
| | | PEEP 10cm $H_2O$ | 9% | -0.04 | 0.05 |
| *Velocity + ABP signal* | ABPmSquaredByPSV | PEEP 10cm $H_2O$ | 5% | 0.08 | 0.06 |
| *Velocity* | SDNN | PLR | 24% | 0.18 | 0.02 |
| | Peak SystolicVelocity | PLR | 8% | 0.09 | 0.04 |
| | VtiPerBeat | PEEP 10cm $H_2O$ | 7% | -0.04 | 0.1 |
| | SystolicFootVelocity | EEOP | 5% | -0.06 | 0.06 |
| | | PEEP 10cm $H_2O$ | 5% | -0.06 | 0 |
| *Velocity + Diameter* | MeanFlowRate Squared | PEEP 10cm $H_2O$ | 8% | 0.01 | 0.06 |
| *Diameter + ABP signal* | ABPpSquaredbyMeanDia | PLR | 10% | 0.11 | 0.01 |
| | | EEOP | 6% | -0.08 | 0.08 |
| *Velocity* | MeanVelocitySquare | PLR | 11% | 0.12 | 0.01 |
| | | PEEP 10cm $H_2O$ | 10% | -0.06 | 0 |
| | IDRVelocity | PLR | 8% | 0.15 | 0.01 |
| | MeanVelocity | PLR | 6% | 0.11 | 0.01 |
| | | PEEP 10cm $H_2O$ | 5% | -0.05 | 0 |

In the above table, 'Velocity Ensemble' refers to the Ensemble average velocity waveform, 'Pressure Ensemble' refers to the ensemble average blood pressure waveform, Diameter refers to the arterial diameter signal, ABP signal refers to a blood pressure signal, 'Velocity' refers to the blood velocity-time signal.

As can be observed from Table 7, all of the features associated with the ensemble average velocity waveform demonstrated statistically significant changes.

More broadly, it was found that ten of the waveform features significantly changed as a result of a PLR, and six as a response to a EEOP test. This demonstrates the particular efficacy of these waveform features in providing indicators or being used to compute indicators of hemodynamic status before and after a clinical intervention event, including fluid responsiveness. For example, the results are suggestive of the potential of these waveform features in monitoring preload responsiveness.

In some embodiments, continuous ultrasound monitoring may be performed, for example using wearable ultrasound sensors, for example a wearable ultrasound patch. Using continuous ultrasound measurements, and the availability of more epochs of data, the discriminatory power of the waveform features may be expected to increase even further. For example, in a setting with the capability to perform continuous, wearable ultrasound measurements, the various waveform features could be used to provide insights to clinicians as to how the patient is responding to an intervention, in real time. Trending analysis might be performed. Furthermore, it is noted that while the study reference above was performed on a cohort of ventilated patients, the efficacy of the various waveform features is not exclusive to this patient population, and can also be applied for spontaneously breathing patients.

As discussed above, embodiments of the invention involve computation of an ensemble average blood velocity waveform from a plurality of blood velocity waveforms extracted from the blood velocity-time signal. This process involves the alignment and averaging of the multiple blood velocity waveforms to produce a single waveform that represents the typical pattern of blood flow velocity over a cardiac cycle.

To compute the ensemble average blood velocity waveform, individual blood velocity waveforms are first identified within the blood velocity-time signal. Each waveform corresponds to a single cardiac cycle and is characterized by a systolic peak followed by a diastolic trough. The waveforms are then aligned with respect to a common feature, such as the onset of systole or the peak systolic velocity, to ensure that the corresponding phases of the cardiac cycle are synchronized across all waveforms.

Once aligned, the waveforms are averaged together to produce the ensemble average waveform. Mathematically, this can be expressed as follows:

$$V_{\text{ensemble}}(t) = \frac{1}{N} \sum_{i=1}^{N} V_i(t)$$

where $V_{\text{ensemble}}(t)$ is the ensemble average blood velocity waveform, $N$ is the number of blood velocity waveforms included in the average, $V_i(t)$ is the blood velocity at time $t$ for the $i$-th waveform, and the summation is performed over the aligned time points $t$ of the cardiac cycle.

The resulting ensemble average waveform provides a stable and representative depiction of the blood velocity waveform, which can be used to compute various waveform features with reduced variability due to physiological fluctuations or measurement noise. For example, the ensemble average waveform can be used to determine the acceleration parameter, which reflects the steepness of the leading edge of the waveform, or the velocity full-width-half-maximum (FWHM) parameter, which provides a measure of the dispersion of the blood velocity around the systolic peak, and/or any combination of one or more of the other waveform features discussed above.

In some cases, the ensemble averaging process may be weighted to account for variations in the quality or reliability of individual waveforms. For instance, waveforms that are less noisy or that have clearer landmark features may be given greater weight in the averaging process. This can be achieved by introducing a set of weights $w_i$ such that:

$$V_{\text{ensemble}}(t) = \frac{\sum_{i=1}^{N} w_i V_i(t)}{\sum_{i=1}^{N} w_i}$$

where $w_i$ is the weight assigned to the $i$-th waveform, and the weights are normalized by their sum to ensure that the ensemble average waveform remains on the same scale as the individual waveforms.

The computation of the ensemble average blood velocity waveform is a robust technique that can enhance the accuracy of hemodynamic parameter estimation by providing a consistent and representative waveform from which to extract clinically relevant features.

The same process outlined above can also be applied for computing an ensemble average blood pressure waveform and/or an ensemble average arterial diameter waveform according to one or more embodiments.

As discussed above, any selected one or more of the various waveform features discussed above can be utilized to determine a hemodynamic response indicator.

With regards to the analysis algorithm which is employed in the method, there are different options available for implementing this. One approach is to use a multiparametric regression function. This is a statistical technique which models the relationship between a dependent variable and multiple independent variables. This type of algorithm could be used to predict the patient's hemodynamic response based on extracted waveform features. This approach has the advantage of permitting a multiplicity of inputs, allowing the computations of the hemodynamic response indicator to be computed in dependence upon a plurality of the waveform features, improving accuracy and reliability.

There will now be outlined a brief explanation of how to train or fit a suitable multiparametric regression model to generate a hemodynamic response indicator based on various input waveform features.

A first step is data collection. This comprises gathering a training dataset comprising a plurality of training dataset entries, wherein each training data entry includes a value for the hemodynamic response indicator and values for each of the waveform features which are intended to be used during operation for generating the hemodynamic response indicator. With regards to the hemodynamic response indicator, this could be a qualitative indicator, i.e. taking one of a closed set of qualitative classifications, e.g. categories such as "responsive," "non-responsive," or "uncertain,". However, alternatively, the hemodynamic response indicator be a quantitative value along a continuous scale or metric.

Once the training dataset has been compiled or acquired, some data pre-processing might be performed, e.g. normalization of the waveform feature values across all of the data entries of the training dataset, or handling any missing data (e.g. imputing or removing missing values). The training dataset may typically be split into training, validation and test data sets, as is standard for training a model or function.

With regards to the regression model which is used, various options are available, such as linear regression model, polynomial regression, or lasso regression.

The model training itself may comprise the following basic steps. The initial model parameters may be configured. The model is then fit to the data. For example, this comprises training the model on the training dataset by minimizing a loss function, typically mean squared error (MSE) for regression tasks. This involves adjusting the model parameters to best predict the hemodynamic response indicator from the waveform features, where the waveform features (or correlates thereof, e.g. normalized versions of the values) are used as the independent parameters of the model. After training, model validation may be performed. The validation is used to tune hyperparameters and avoid overfitting. Techniques such as cross-validation may be used.

Finally, a step of model evaluation may be performed. This comprises evaluating the model using a test dataset to assess its performance. Common metrics for evaluating performance include MSE, mean absolute error (MAE), and $R^2$ (coefficient of determination). Residual analysis may be performed which comprises checking identifying whether any patterns exist in the residual values computed when determining the chosen metric of error, which might suggest poor model fit.

Once trained, the regression function can be applied to new patient data in real time. The function may be trained to receive as input variables values for the first set of parameters 24, output a first value of the hemodynamic response indicator corresponding to the first epoch, and then separately receive as input variables values for the second set of parameters 26 to output a second value of the hemodynamic response indicator corresponding to the second epoch. The first set of parameters and second set of parameters contain values for the same set of waveform features. By repeatedly acquiring new values for the set of parameters and monitoring how the hemodynamic response indicator changes, clinicians can gain insights into the patient's fluid responsiveness and other aspects of hemodynamic status.

Another approach is to implement a machine learning algorithm, which can be particularly advantageous due to its ability to learn from data and make predictions or decisions without being explicitly programmed to perform the task. Machine learning algorithms can include, but are not limited to, support vector machines, decision trees, neural networks, and ensemble methods like random forests.

To train a machine learning algorithm, a dataset comprising previous patient data is used, where each data entry in the training data includes the extracted waveform features and the known hemodynamic response for a given patient. The algorithm learns to map the features to the hemodynamic response through a process that involves adjusting the algorithm's parameters to minimize the difference between the predicted and actual responses. This training process may utilize techniques such as cross-validation to ensure that the model generalizes well to unseen data.

Once trained, the machine learning algorithm can be applied to new patient data in real time. The algorithm may be trained receive the first set of parameters 24 as input, output a first value of the hemodynamic response indicator corresponding to the first epoch, and then separately receive the second set of parameters 26 to output a second value of the hemodynamic response indicator corresponding to the second epoch. By repeatedly acquiring new values for the set of parameters and monitoring how the hemodynamic response indicator changes, clinicians can gain insights into the patient's fluid responsiveness and other aspects of hemodynamic status.

For example, the machine learning algorithm might classify the patient's hemodynamic responsiveness into categories such as "responsive," "non-responsive," or "uncertain," based on the features extracted from the ensemble average velocity waveform. The classifications could be used to guide clinical decisions, such as whether to administer fluids or adjust other aspects of patient care. Alternatively, the hemodynamic response indicator may be numerical value.

In some embodiments, the analysis algorithm may comprise a classifier algorithm or model. This can be implemented using a regression model, a machine learning model (such as a neural network) or any other suitable type of model. A classifier is a type of machine learning model that is used for categorical prediction. The goal of a classifier is to assign class labels to instances based on the input features. These class labels are discrete, and there are a finite number of them. Common examples include: binary classification, or multiclass classification. A machine learning model such as a neural network can be trained to provide a classification as an output. A linear regression model can also be used to provide a classification output, for example by designating classification labels to different ranges of values of the output from the model.

In some embodiments, the analysis algorithm comprises a plurality of classifiers which may be used as an ensemble in generating a single final output hemodynamic response indicator. For example, a separate respective classifier might be trained for each of a plurality of different of the waveform features, to generate a respective output classification relating to each of the plurality of waveform features and wherein the plurality of outputs from the ensemble of classifiers are then used to compute a final output hemodynamic response indicator, e.g. using another classifier or function or any other method.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A computer-implemented method, comprising:

   - receiving (12) first ultrasound data of a blood vessel of a subject, the first ultrasound data acquired over a first epoch, the first epoch occurring prior to a clinical intervention event;
   - receiving (14) second ultrasound data of a blood vessel of a subject, the second ultrasound data acquired over a second epoch, the second epoch occurring following the clinical intervention event;
   - deriving (16) from the received first ultrasound data a first blood velocity-time signal representative of blood velocity over a time window contained in the first epoch, and computing (20) a first ensemble average blood velocity waveform from a plurality of blood velocity waveforms present in the first blood velocity-time signal;
   - deriving (18) from the second ultrasound data a second blood velocity-time signal representative of blood velocity over a time window contained in the second epoch, and computing (22) a second ensemble average blood velocity waveform from a plurality of blood velocity waveforms present in the second blood velocity-time signal;

- determining (24) a first set of parameters, the determining the first set of parameters including extracting from the first ensemble average blood velocity waveform one or more pre-defined blood velocity waveform features;
- determining (26) a second set of parameters, the determining the second set of parameters including extracting from the second ensemble average blood velocity waveform the one or more pre-defined blood velocity waveform features;
- retrieving and applying a pre-defined analysis algorithm, the analysis algorithm configured to receive said first and second sets of parameters as inputs and to generate (28) a hemodynamic response indicator as an output, the hemodynamic response indicator indicative of characteristics of a patient's hemodynamic responsiveness to the clinical intervention event.

2. The method of claim 1, wherein the analysis algorithm comprises a classifier algorithm and wherein the hemodynamic response indicator output from the analysis algorithm is a classification of patient hemodynamic responsiveness into one of a pre-defined set of possible classifications.

3. The method of claim 1 or claim 2, wherein the analysis algorithm comprises a machine learning algorithm.

4. The method of claim 1, further comprising:

   - deriving from the first ultrasound data a first arterial diameter signal representative of a diameter of the blood vessel over said time window contained in the first epoch;
   - deriving from the second ultrasound data a second arterial diameter signal representative of a diameter of the blood vessel over said time window contained in the second epoch; and
   - wherein the computing the first set of parameters further comprises extracting from the first arterial diameter signal one or more pre-defined arterial diameter waveform features;
   - wherein the determining the second set of parameters further comprises extracting from the second arterial diameter signal the one or more pre-defined arterial diameter waveform features.

5. The method of any preceding claim, wherein the clinical intervention event is one or more of: end-expiratory occlusion pressure, inspiratory hold positive end-expiratory pressure 10cm H2O, inspiratory hold positive end-expiratory pressure 15cm H2O, a passive leg raise, and intra-arterial administration of a bolus of liquid to the subject.

6. The method of any preceding claim, wherein each of the first ultrasound data and second ultrasound data comprise both B-mode ultrasound data and pulsed wave Doppler ultrasound data, and preferably wherein the blood velocity-time signal is derived using the pulsed wave Doppler data.

7. The method of any of claims 1-6, further comprising obtaining first and second blood pressure measurement information for the subject corresponding to the first and second epochs and wherein the analysis algorithm is further configured to receive the first and second blood pressure measurement information as inputs.

8. The method of claim 7, wherein the obtaining the first and second blood pressure measurement information comprises obtaining first and second blood pressure signals for the subject indicative of a blood pressure waveform for the subject over the time window contained in the first and second epochs respectively.

9. The method of claim 8,

   wherein the method comprises computing a first ensemble average blood pressure waveform from a plurality of blood pressure waveforms present in the first blood pressure signal and further computing a second ensemble average blood pressure waveform from a plurality of blood pressure waveforms present in the second blood pressure signal; and
   wherein the first and second blood pressure measurement information is derived from the first and second ensemble average blood pressure waveforms.

10. The method of claim 9,

   wherein the determining the first set of parameters includes extracting from the first ensemble average blood pressure waveform one or more pre-defined blood pressure waveform features, and
   wherein the determining the second set of parameters includes computing from the second ensemble average blood pressure waveform the same one or more pre-defined blood pressure waveform features;

wherein the blood pressure waveform features extracted from the first and second ensemble average blood pressure waveforms provide the blood pressure measurement information.

11. The method of any of claims 1-10, further comprising:

- deriving from the first ultrasound data a first arterial diameter signal representative of a diameter of the blood vessel over said time window contained in the first epoch, and computing a first ensemble average arterial diameter waveform from a plurality of arterial diameter waveforms present in the first arterial diameter signal;
- deriving from the second ultrasound data a second arterial diameter signal representative of a diameter of the blood vessel over said time window contained in the second epoch and computing a second ensemble average arterial diameter waveform from a plurality of arterial diameter waveforms present in the second arterial diameter signal; and
- wherein the computing the first set of parameters comprises computing from the first ensemble average diameter waveform one or more pre-defined arterial diameter waveform features and computing from the second ensemble average diameter waveform the same one or more pre-defined arterial diameter waveform features.

12. The method of any of claims 1-11, wherein each of the first and second set of parameters comprise a same group of waveform features which include at least one of:

skewness of the ensemble average velocity waveform;
kurtosis of the ensemble average velocity waveform;
Full Width Half Maximum, FWHM, of the ensemble average velocity waveform; and
an acceleration of the ensemble average velocity waveform indicative of a steepness of a leading edge of the ensemble average velocity waveform.

13. A computer program product comprising computer code configured to cause a processor to perform a method in accordance with any of claims 1-12.

14. A processing device comprising one or more processors configured to perform a method in accordance with any of claims 1-12.

15. A system (30), comprising;
the processing device (32) of claim 14; and
an ultrasound acquisition apparatus (54) for acquiring the first and/or second ultrasound data.

10 ⟋⟍

Receive 1ˢᵗ U/S data  ⟋12

↓

Derive 1ˢᵗ blood velocity signal  ⟋16

↓

Compute 1ˢᵗ ensemble average velocity WF  ⟋20

↓

determine 1ˢᵗ parameter set  ⟋24

Receive 2ⁿᵈ U/S data  ⟋14

↓

Derive 2ⁿᵈ blood velocity signal  ⟋18

↓

Compute 2ⁿᵈ ensemble average velocity WF  ⟋22

↓

determine 2ⁿᵈ parameter set  ⟋26

↓

Compute hemodyn. response indicator  ⟋28

**FIG. 1**

⟋32

34 ⟋

I/O

38 ⟋

Memory

proc  ⟋36

42 ⟋

B-mode data

PWD data

⟋44

U/S Apparatus  ⟋54

30

**FIG. 2**

FIG. 3

FIG. 4

FIG. 5

**During PLR**

FIG. 6

| | Europäisches Patentamt European Patent Office Office européen des brevets | **EUROPEAN SEARCH REPORT** | **Application Number** EP 24 21 0947 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 4 342 387 A1 (KONINKLIJKE PHILIPS NV [NL]) 27 March 2024 (2024-03-27) * paragraphs [0003], [0018], [0043], [0045] - [0047], [0058], [0061], [0065], [0068]; figures 1,3 * ----- | 1,2,4-8, 13-15 | INV. A61B8/06 A61B8/08 A61B8/00  ADD. A61B8/04 |
| Y | US 2023/210491 A1 (JOSHI ROHAN [NL]) 6 July 2023 (2023-07-06) * paragraph [0082] * ----- | 1,2,4-8, 13-15 | |
| A | EP 4 378 394 A1 (KONINKLIJKE PHILIPS NV [NL]) 5 June 2024 (2024-06-05) * the whole document * ----- | 1-15 | |

**TECHNICAL FIELDS SEARCHED (IPC)**

A61B

The present search report has been drawn up for all claims

| Place of search | Date of completion of the search | Examiner |
|---|---|---|
| Munich | 8 April 2025 | Koprinarov, Ivaylo |

EPO FORM 1503 03.82 (P04C01)

## ANNEX TO THE EUROPEAN SEARCH REPORT
## ON EUROPEAN PATENT APPLICATION NO.

EP 24 21 0947

08-04-2025

| Patent document cited in search report | | Publication date | Patent family member(s) | | Publication date |
|---|---|---|---|---|---|
| EP 4342387 | A1 | 27-03-2024 | EP | 4342387 A1 | 27-03-2024 |
| | | | WO | 2024061607 A1 | 28-03-2024 |
| US 2023210491 | A1 | 06-07-2023 | CN | 115715170 A | 24-02-2023 |
| | | | EP | 4164501 A2 | 19-04-2023 |
| | | | JP | 2023528679 A | 05-07-2023 |
| | | | US | 2023210491 A1 | 06-07-2023 |
| | | | WO | 2021250234 A2 | 16-12-2021 |
| EP 4378394 | A1 | 05-06-2024 | NONE | | |

EPO FORM P0459